# EUROPEAN PATENT SPECIFICATION

(11) **EP 4 183 354 B1**
(45) Date of publication and mention of the grant of the patent: **03.09.2025**
(21) Application number: 22205896.8
(22) Date of filing: 07.11.2022
(51) Int. Cl.: A61B 17/17, A61B 90/00, A61B 90/11, A61B 17/16, A61B 17/32

(54) **OFFSET GUIDE**
OFFSET-FÜHRUNG
GUIDE DÉCALÉ

(30) Priority: 12.11.2021 US 202163278692 P; 02.03.2022 US 202263315595 P
(43) Date of publication of application: 24.05.2023
(73) Proprietor: Smith & Nephew Asia Pacific Pte. Limited, Singapore 138565 (SG); Smith & Nephew Orthopaedics AG, 6300 Zug (CH); Smith & Nephew, Inc., Memphis, TN 38116 (US)
(72) Inventor: HOUSMAN, Mark Edwin, Memphis, 38116 (US); HALL, Benjamin Michael, Mansfield, 02048 (US); PATEL, Nethal Navinbhal, Mansfield, 02048 (US); BALBOA, Marc J, Massachusetts, 01748 (US)
(74) Representative: Appleyard Lees IP LLP

(56) References cited:
- WO-A1-2005/051209
- WO-A1-2019/027821
- US-A1- 2010 049 198
- US-A1- 2012 253 352
- US-A1- 2021 015 504

## Description

### FIELD

The present disclosure relates to guides for tissue repair. More specifically it discloses a guide with a guide arm that engages a bone and places a tool through the bone at a preferred location along the bone.

### BACKGROUND

Reconstructive surgery for joints such as arm and leg joints often involve surgical attachments that are subject to substantial force due to the pivotal movement these joints provide and weight of the limbs themselves. Reconstructive surgery involving functional, structural fixation to bones of a joint may involve drilling into at least one of these bones. This may form a passage within the bone for fixation constructs including flexible connecting members and anchors to extend through and/or anchor within. In the case of an acromioclavicular joint repair, the surgery may employ a tunnel guide with a drilling locator to facilitate compression/reduction of a joint to place the two bones (clavicle and coracoid) in a target arrangement relative to each other while drilling. The drill locator aligns the drill along and through the axis of reduction for repair of the acromioclavicular (AC) and/or coracoclavicular (CC) joint. A drill may extend along the axis of reduction until it meets a distal hard stop feature of the guide at an inferior aspect of the coracoid.

The coracoid however is a fragile arch shaped process of bone, with a variable cross section, that crisscrosses under the clavicle. The arch shape has an apex portion that defines a stable location for a guide arm to hold the coracoid and position the coracoid and clavicle in the target arrangement (reduced configuration) and thereby the apex portion may define one end of the reduction axis. Guides known in the art that hold a clavicle and coracoid in a reduced configuration guide a drill along an axis that is coincident with the guide arm location and therefore through the apex portion of the coracoid. This portion however tends to be thinner in cross section than other portions of the coracoid, and therefore less preferable a location to drill a hole therethrough. Moving the guide arm distal end along the coracoid towards these more preferably locations for drilling, however, may frustrate a stable hold on the coracoid, as a result of the arch shape of the coracoid. There is therefore a need for a guide that holds the coracoid and clavicle in a reduced configuration, accommodating for the coracoid anatomy relative to the clavicle. There is also a need for a guide that guides a drill through the coracoid, along a trajectory that defines a subsequent fixation location through a portion of the coracoid with sufficient cross section, to reduce a risk of fracture.

There may also be risk of coracoid fracture, should the drill form a passage offset from a center of the coracoid cross section. The coracoid is a relatively thin process of bone. Guides known in the art may guide a drill along an axis coincident with the guide arm location but depending on the shape of the guide arm and shape of the coracoid cross section, the trajectory may be offset or angled away from a center of the coracoid cross section and risk fracture thereof. Fracture may occur during drilling or later one, via uneven stresses from the fixation construct. There is therefore a need for a guide that holds the coracoid and clavicle in an anatomically reconstructed arrangement, centering the drill trajectory through the coracoid cross section.

US 2021/0015504 A1 describes a coracoid drill guide assembly that includes a body having a cylindrical channel on each side of the body for receiving a drill guide sleeve. US 2010/0049198 A1 describes a device for positioning a tibial tunnel during ACL reconstruction. WO 2019/027821 A1 describes a targeting guide that includes a body defining a first guide hole sized and configured to receive a guide sleeve therethrough. US 2012/253352 A1 describes an acromioclavicular joint guide for forming multiple drill tunnels spaced apart by a predetermined distance employs two drill guides in a pivoting arrangement allowing one drill guide to pivot around the axis of the other. WO 2005/051209 A1 describes a guide clamp for clamping a femur and guiding placement of a guide wire relative to the femur.

### SUMMARY

An offset guide for holding a surgical joint in a reduced configuration in accordance with the invention is defined in claim 1. Further embodiments of the invention are recited in the dependent claims. Disclosed herein is a drill guide that locates a drill at a location offset from an axis of reduction. The guide may engage a bone outer surface at a location that is stable for gripping and holding the bone for stable joint reduction, while placing a drill hole through the same bone in an offset location therefrom. The offset location may be along a portion of the bone that has a larger cross section and therefore is less likely to fracture while drilling a hole therethrough. The hole is drilled to aid in coupling a repair construct to the bone, and the larger cross section may be better able to withstand the clinical loading requirements transmitted via the repair construct. This offset location may also provide improved visualization of the drill and/or repair construct implants, as they exit the bone at the offset location spaced away from the reduction axis. This offset may also provide space for an implant to deploy outside the bone without encountering the guide. Deploying may include expanding laterally and towards the reduction axis. This offset is also sufficient to avoid metal to metal contact between the drill and the guide, reducing the generation metal debris inside the body.

Disclosed herein is a drill guide that centers a drill through the coracoid for a range of coracoid cross sections. The guide may wrap around three side surfaces of the bone outer surface at a location that is stable for gripping the bone for joint reduction. The guide may define a V-shaped opening that engages two discrete surface portions of the bone, on opposite sides of the bone. The V-shaped opening may center the bone within the V-shaped opening. The drill may then form a passage through a center of the bone. This passage may be offset from the V-shaped opening. The offset location may be along a portion of the bone that has a larger cross section and therefore is less likely to fracture while drilling a hole therethrough. The hole is drilled to aid in coupling a repair construct to the bone, and the larger cross section may be better able to withstand the clinical loading requirements transmitted via the repair construct. This offset location may also provide improved visualization of the drill and/or repair construct implants, as they exit the bone at the offset location spaced away from the reduction axis. This offset may also provide space for an implant to deploy outside the bone without encountering the guide. Deploying may include expanding laterally and towards the reduction axis. This offset is also sufficient to avoid metal to metal contact between the drill and the guide, reducing the generation metal debris inside the body.

Disclosed herein is an offset guide for holding a surgical joint in a reduced configuration. The offset guide is configured to place a fixation construct in an offset location relative to the axis of reduction. The offset guide may include a proximal end, a distal end and a curved arm extending therebetween, the curves arm extending around the joint. The distal end engages a bone of the surgical joint at a first location along the bone to as to apply a load to the bone to hold the bone in the reduced configuration. The proximal end includes a guiding means that aims placement of a surgical tool through the bone towards an offset location along the bone, the offset location spaced away from the first location. The offset location is axially spaced from the first location. The offset location is a portion of the bone that has a larger cross section than a corresponding cross section of the first location.

In some offset guide embodiment, the offset location may be through a portion of the bone that frustrates engagement with the guide distal end, and more specifically the offset location frustrates applying a load at that location that effectively reduces the surgical joint. The surgical tool may include a drill tool and then an implant insertion tool, the implant a part of a fixation construct. The distal end may be offset by at least 5mm from the offset location. The guide distal end may engage an apex of a coracoid bone and the offset location may be a segment of the coracoid bone proximally or medially disposed relative to the apex. The guide distal end may be formed in a hook that wraps around three sides of the bone and self-centers the distal end relative to the bone such that the surgical tool is placed through a center of the bone at the offset location.

Another example guide is disclosed, that guides formation of a passage through a first and second bone at a target location. The example guide includes a proximal end, a distal end and a curved arm extending therebetween. The guide proximal end includes a channel having a longitudinal axis that defines a passage trajectory through a bone thickness of both a first and a second bone. The distal end is formed in a hook sized and shaped to wrap around three sides of the first bone to self-center the guide distal end relative to the first bone, such that the passage trajectory extends through the target location which includes through a center of the first bone.

Some distal end hooks may include two opposing surfaces, facing each other and wherein the two opposing surfaces are configured to engage, one each, a first and a second side surface of the first bone three sides, the first and second sides defining opposing sides of the first bone. The guide distal end may wrap around three sides of an apex of a coracoid and the passage trajectory may place a surgical tool through a location through the coracoid posteriorly offset from the apex. Some example guides define a passage trajectory that extends through the center of the first bone at an offset location along an axis of the first bone, from the guide distal end. The channel may sequentially receive a drill tool and an implant insertion tool therethrough. The passage trajectory through the bone thickness of the first and the second bone may place the passage at an offset location through the first bone, the offset location a location that frustrates engagement and joint reduction with the guide distal end. The offset location may be at least 5mm from the hook along the first bone. The guide distal end may engage an apex of a coracoid bone and the offset location may be a portion of the coracoid bone medially disposed relative to the apex.

An example method for locating an insertion site for a repair of a surgical joint is also disclosed herein. The method may include engaging an outer surface of a first bone of the surgical joint with a distal end of an offset guide, defining a first engagement location and engaging an outer surface of a second bone of the surgical joint with a proximal end of the offset guide defining a second engagement location, and thereby defining a reduction axis that extends through the first and second engagement locations. While engaging the outer surfaces of the first and second bones the method may include inserting a surgical tool through a guide sleeve of the offset guide proximal end, and then through the outer surfaces of the first bone at an offset location, spaced away from the first engagement location.

Engaging the outer surfaces may hold the first and second bones in a reduced arrangement. The surgical tool may be an implant delivery device and inserting may also include placing an implant through the first and second bone and placing the implant at the offset location, while the offset guide remains engaged with the outer surfaces. The outer surface of the first bone may be an inferior surface of a coracoid, and the outer surface of the second bone may be a superior surface of a clavicle. The first location may be an apex of an arched portion along a coracoid and the offset location may be disposed along the coracoid and towards a base of the coracoid. The method may also include observing the surgical tool as it extends through the first bone outer surface, the offset guide second end spaced away from the offset location to provide a clear view thereof.

These and other features and advantages will be apparent from a reading of the following detailed description and a review of the associated drawings. It is to be understood that both the foregoing general description and the following detailed description are explanatory only and are not restrictive of aspects as claimed.

### BRIEF DESCRIPTION OF THE DRAWINGS

The disclosure will be more fully understood by reference to the detailed description, in conjunction with the following figures, wherein:
FIG. 1A illustrates the anatomy of the shoulder for reference purposes;
FIG. 1B illustrates a superior surface of the coracoid for reference purposes;
FIG. 2A illustrates the guide and drill components of the offset guide system, in accordance with this disclosure;
FIG. 2B illustrates the guide with the drill inserted along the guide sleeve, in accordance with this disclosure;
FIG. 2C illustrates an end view of the guide with the drill sleeve inserted along the guide sleeve, in accordance with this disclosure;
FIG. 3A illustrates the offset guide engaged on a coracoid with the drill sleeve inserted through the coracoid at a location offset from the guide distal end, in accordance with this disclosure;
FIG. 3B illustrates a close-up of the offset guide components through the coracoid, in accordance with this disclosure;
FIG. 3C illustrates a close-up of the offset guide engaging the coracoid apex, and observing an anchoring implant through the offset tunnel, in accordance with this disclosure;
FIG. 4A illustrates a bone cross section with a passage extending through the center, for illustrations purposes;
FIG. 4B illustrates a bone cross section with a passage extending through off-center, for illustrations purposes;
FIG. 5A illustrates a hook end of a drill guide, known in the art relative to a bone cross section;
FIG. 5B illustrates a hook end of a drill guide, known in the art relative to another bone cross section;
FIG. 6A illustrates a V-shapes drill guide distal aspect engaging two opposing sides of a bone, in accordance with this disclosure;
FIG. 6B illustrates a V-shaped drill guide distal aspect, in accordance with this disclosure;
FIG. 7A illustrates a drill guide and drill with a V-shaped drill guide distal aspect, in accordance with this disclosure;
FIG. 7B illustrates the V-shaped drill guide distal aspect, in accordance with this disclosure; and
FIG. 7C illustrates a V-shaped drill guide distal aspect that is offset from the drill trajectory, in accordance with this disclosure.

### DETAILED DESCRIPTION

In the description that follows, like components have been given the same reference numerals, regardless of whether they are shown in different examples. To illustrate example(s) in a clear and concise manner, the drawings may not necessarily be to scale and certain features may be shown in somewhat schematic form. Features that are described and/or illustrated with respect to one example may be used in the same way or in a similar way in one or more other examples and/or in combination with or instead of the features of the other examples.

As used in the specification and claims, for the purposes of describing and defining the invention, the terms "about" and "substantially" are used to represent the inherent degree of uncertainty that may be attributed to any quantitative comparison, value, measurement, or other representation. The terms "about" and "substantially" are also used herein to represent the degree by which a quantitative representation may vary from a stated reference without resulting in a change in the basic function of the subject matter at issue. "Comprise," "include," and/or plural forms of each are open ended and include the listed parts and can include additional parts that are not listed. "And/or" is open-ended and includes one or more of the listed parts and combinations of the listed parts. Use of the terms "upper," "lower," "upwards," and the like is intended only to help in the clear description of the present disclosure and are not intended to limit the structure, positioning and/or operation of the disclosure in any manner.

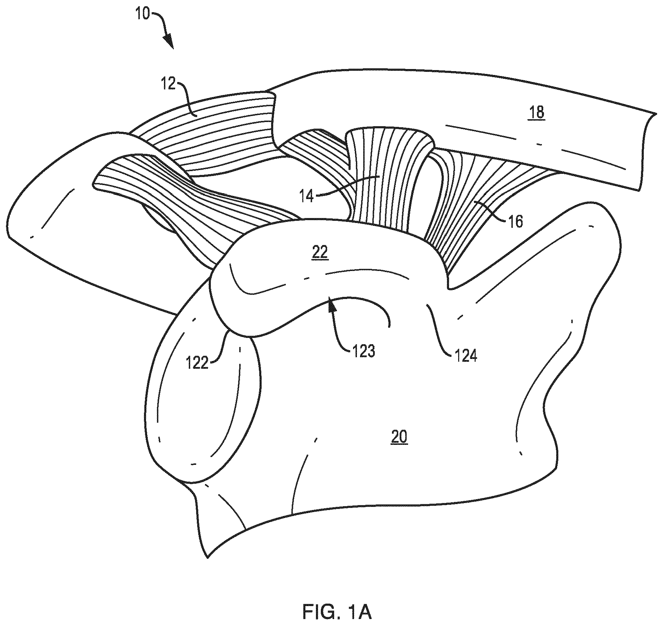

FIG. 1A illustrates the anatomy of the shoulder joint 10, for reference purposes. FIG. 1A illustrates the AC ligament 12, trapezoid ligament 14 and conoid ligament 16, all of which may be damaged or torn. During an AC joint repair, the clavicle 18 may be reattached to the coracoid process 22 of the scapula 20 (hereinafter "coracoid"). During the repair, the clavicle 18 may be relocated relative to the coracoid 22. This is oftentimes referred to as reducing the joint or placing the joint in a reduced configuration. During the repair, a repair construct may be coupled to both the clavicle 18 and coracoid 22 to hold them in the reduced configuration. This may involve drilling holes through the clavicle 18 and coracoid 22 to receive portions of the repair construct therethrough. An example repair of an AC joint is illustrated in U.S. Patent 10,123,813.

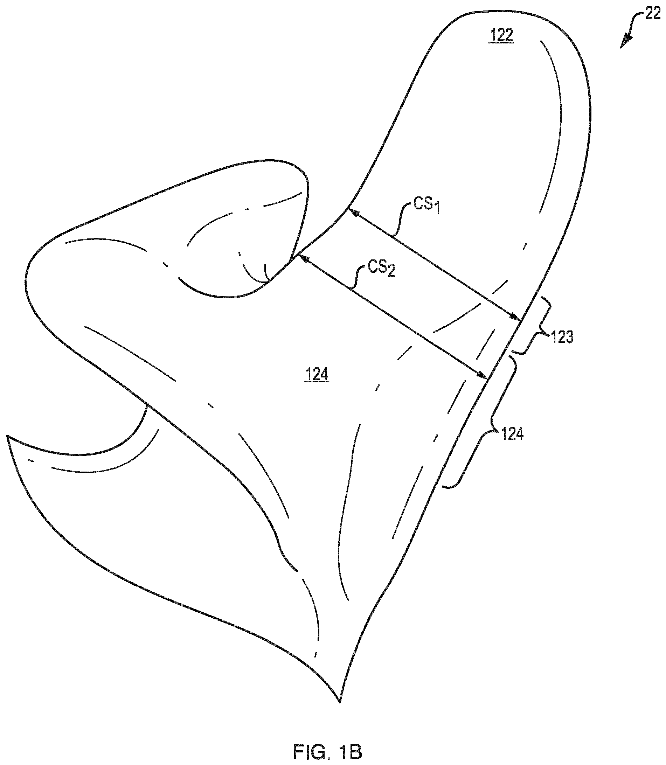

The coracoid 22 is an arch shaped projection with segments including a tip 122, an arched midportion including an apex 123 and a base 124. The coracoid 22 is relatively small and fragile, and drilling holes has been known to compromise the coracoid 22. The base 124 is posterior and medial to the tip 122 and apex 123. The coracoid 22 crisscrosses under the clavicle 18 and therefore in an anatomical reconstruction, the clavicle 18 and coracoid 22 may vertically align with each other for only a short segment of each bone. This may be approximately along the apex 123 of the coracoid 22 and may be adjacent attachment points of trapezoidal ligament 14. The coracoid base 124 gradually broadens as it extends posteriorly and is attached by a broad base to the upper part of the neck of the scapula. The apex 123 is generally located along a segment of the coracoid 22 that is smaller in cross section than the base 124. FIG. 1B illustrates a top-down view of the coracoid 22, with the clavicle 18 removed. Illustrated is the cross-sectional width CS1 at the apex 123 relative to the cross-sectional width CS2 closer to the base 124.

Advantageously, the offset guide 100 disclosed herein allows the user to grip the anatomy, in this case the inferior surface of the coracoid 22, in a "low energy" portion of the coracoid 22. This may be the apex 123 of the arched midportion of the coracoid 22. The apex 123 may stably nest a guide distal end. The offset guide 100 however inserts a drill along a trajectory that extends through the coracoid 22 at a more optimal location along the coracoid 22, for forming passages therethrough, and therefore spaced away from the apex 123. This offset location is preferably proximal and posterior to the apex 123. This offset location is closer to the base portion 124 of the coracoid 22. The proximal posterior base portion 124 is larger in width, and therefore preferable for placing a hole or tunnel therethrough, as explained herein.

The offset guide 100 allows the user to observe the drill tip and/or implantable device of the repair construct as it exits the inferior surface of the coracoid 22. Stated another way, the guide distal end preferably does not obscure or obstruct visualization of the drill and implant/anchor as it exits the bone tunnel. The offset guide 100 allows space for an implantable device to deploy outside the bone without encountering the guide. Deployment oftentimes involves changing the implantable device to a laterally expanded configuration or flipping the implant to a larger footprint to engage the outer cortical surface of the bone. The offset is preferably sufficient to avoid interference between the deployed implantable device and guide distal end. This allows the offset guide 100 to remain engaged with the coracoid 22, maintaining the joint in the reduced configuration, while fixing the joint with the repair construct, including deploying the implant/anchor. Offsets may be between 5-10mm between the guide arm that engages the coracoid inferior surface to hold the coracoid 22, and the drill location through the coracoid inferior surface.

Offset guide 100 disassociates the drill stop mechanism from the distal aspect of the guide. This is advantageous as the drill avoids the distal aspect of the guide at the coracoid inferior surface, and therefore avoids metal to metal contact between the spinning drill point and the guide, which can generate metal debris inside the body.

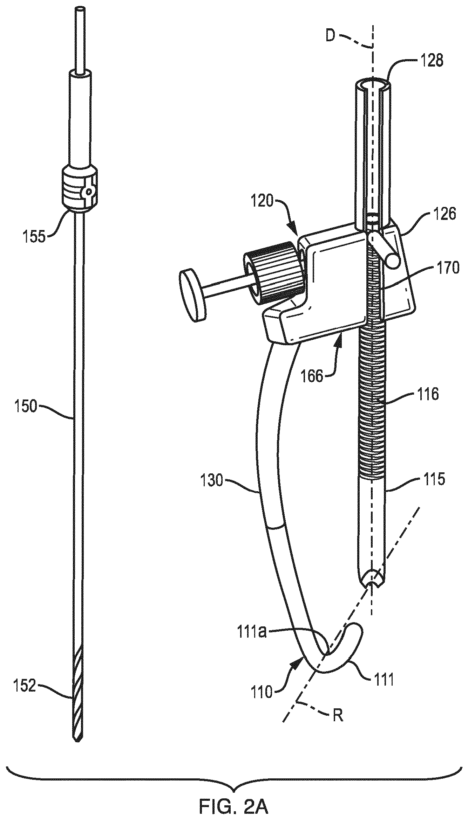

FIG. 2A illustrates an embodiment of a system including an offset guide 100 and drill 150. The offset guide 100 includes a guide distal aspect or distal end 110 and proximal end 120. A curved arm 130 may extend therebetween, to wrap around the target joint anatomy. Distal end 110 defines a finger 111 defining a proximal facing surface 111a configured to wrap around and engage an inferior surface of coracoid 22, the point or area of engagement defining one end of the reduction axis R-R. Proximal end 120 may include a housing 126 with a distal surface 166 for engaging the shoulder of the patient. Housing 126 also defines an elongate opening 170 for receiving bullet 115. Bullet 115 may be axially slideable along elongate opening 170 and controlled with ratchets or pawls 116. Distal end 118 of bullet 115 may be axially advanced, to engage a superior surface of clavicle 18. This defines a second end of the reduction axis R-R, and bullet distal end 118 and finger 111 are configured to hold the two bones (coracoid and clavicle) in a target reduced configuration while attached a fixation construct.

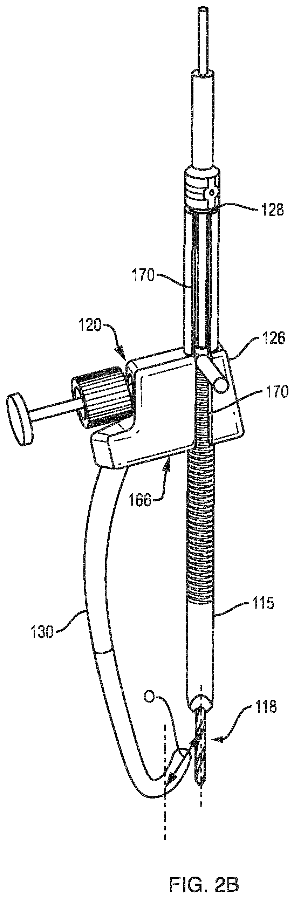

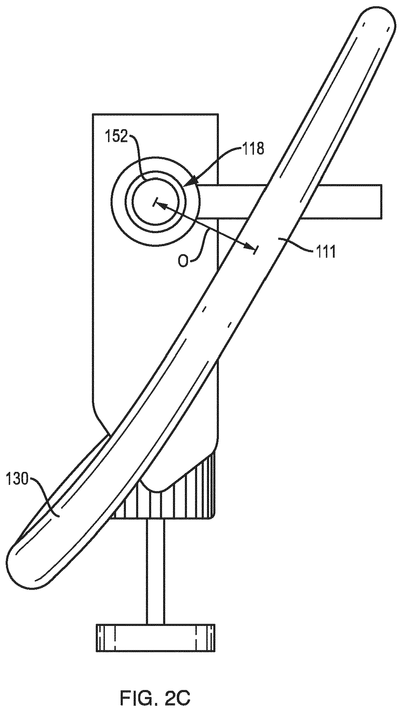

Seen in FIG. 2B, elongate opening 170 and bullet 115 both extend along an axis (D-D) that is angularly offset from reduction axis R-R, to define the offset location through the coracoid 22. Bullet 115 may be cannulated, to receive surgical tools therethrough. For example, a wire, or drill 150 may be inserted therethrough. Drill 150 may be a sleeved drill to extend over wire. Bullet 115 therefore defines the trajectory of drill tip 152 through the clavicle 18 and coracoid 22, along axis D-D. Bullet 115 therefore also defines an axis that places the drill through the coracoid 22 at an offset location relative to the guide finger 111. Offset "O" may be seen in FIG. 2B and FIG. 2C. Drill 150 may include a drill stop 155 configured to limit the insertion depth of drill tip 152 through the coracoid 22. Drill stop surface 128 on guide 100 is sized to interact with drill stop 155 and limit the depth of insertion. FIG. 2C illustrates a bottom view of guide 100 with drill stop 152 inserted. FIG. 2A-2C illustrate a right sided guide system, for the right AC joint of a patient. A mirror image guide may also be provided where the arrangement of the bullet and guide arm are a mirror image for the patient's left side.

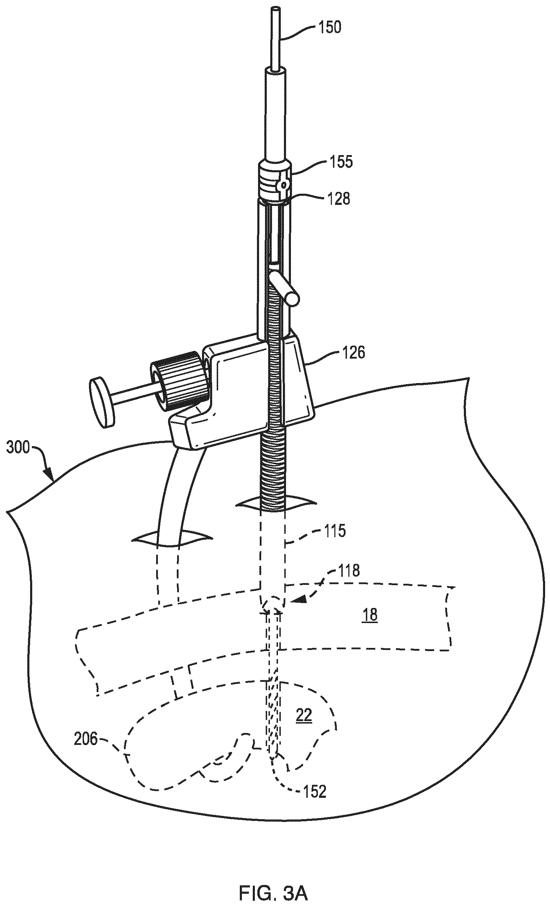

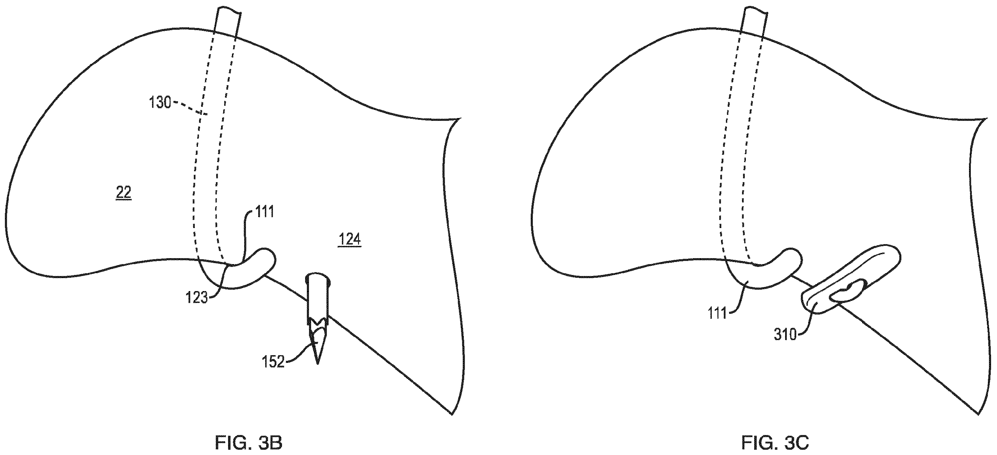

FIGS. 3A-3B shows the guide and drill (right sided) assembled to the AC joint. Guide housing 126 may be positioned on top of the patient shoulder 300. Guide arm 130 may be positioned through at least one patient portal and finger 111 may be wrapped around and under the coracoid 22. More specifically, guide finger 111 engages apex 123 of coracoid, best seen in FIG. 3B. Bullet 115 is inserted through elongate opening 170 of housing and through another patient portal to engage a superior surface of clavicle 18. The two bones are held in position relative to each other via bullet end 118 and finger superior surface 111a. Drill tip 152 is shown inserted through cannulated bullet 115 and forms a passage through both the clavicle 18 and coracoid 22, the passage through coracoid 22 at an offset location relative to finger 111 and thereby offset from apex 123 of the coracoid 22. Offset location is posterior and towards the coracoid base 124. Drill tip 152 may be inserted until drill stop 155 abuts drills top surface 128. Drill may include a sleeved drill. A guide wire may first form a passage through the bones and a sleeve drill may enlarge the passage diameter. FIG. 3B illustrates a close-up of finger 111 engaged with apex 123. Drill tip 152 has formed a passage through coracoid 22 closer to the base 124 of coracoid 122. Resulting passage has a diameter configured to cooperate with a repair construct and preferably has as small a diameter possible to save coracoid bone tissue. Passage diameter is preferable smaller than a deployed footprint size of an implant/anchor of fixation construct. Illustrated in FIG. 3C, the drill may be removed and an anchor 310 may be inserted through passages (clavicle and/coracoid) and deployed to anchor repair construct to the coracoid 22, while the guide arm 130 and finger 111 are still engaged with the joint. This helps to keep the joint in the reduced configuration while the repair construct is being assembled.

In some embodiments, a drill stop may be avoided, as the surgical tools are readily observed as they exit the coracoid 22. In some embodiments the proximal end may include several elongate passages similar to 170, at a variety of locations or angles for different offset locations. In some embodiments the elongate passage may be articulable, to adjust the offset location.

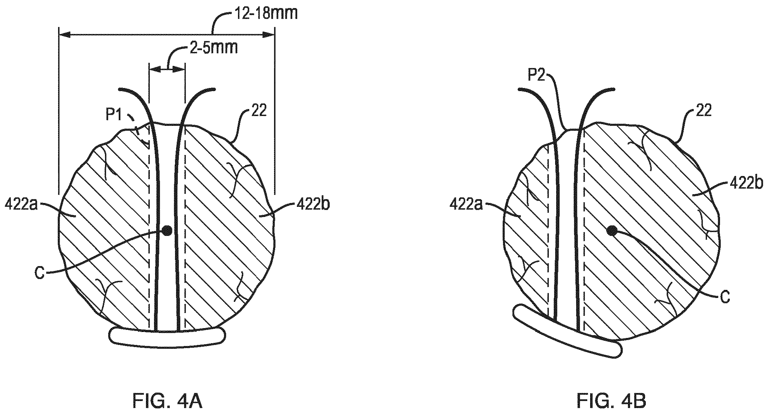

In some embodiments, the distal aspect of the AC joint guide may be configured to center the drill trajectory relative to the target bone. Referring to previous figures, finger 111 may be shaped to center the drill trajectory. FIG. 4A and 4B schematically show two example cross sections of a coracoid 22 with example passages that may be formed therethrough. FIG. 4A illustrates a centered passage P1 therethrough. FIG. 4B illustrates an example off-centered passage P2 therethrough. Coracoid cross section is represented in these figures as approximately circular. It may be more oblong, or egg shaped, and FIGS. 4A-4B are illustrative, for discussion purposes only. Coracoid 22 may have a cross section width between 12-18mm. Consider now that a passage formed therethrough during an AC joint repair may be 2-5mm is diameter. This not only is a substantial portion of the coracoid cross section, which is considered a fragile bone to begin with, but should the passage be offset (FIG. 4B) from the center "C", one side 422a may be considerable smaller still. This may risk coracoid fracture either while drilling or under load. In addition, when placed off center, the anchor may be positioned at a location that risks repair failure. For example, the anchor may gain insufficient footprint purchase with the coracoid 22 causing higher stress loading on the target bone. Furthermore, the resulting force vector between the coracoid 22 and clavicle 18 with an anchor offset from the bone center may draw the two bones out of the anatomical reconstruction arrangement.

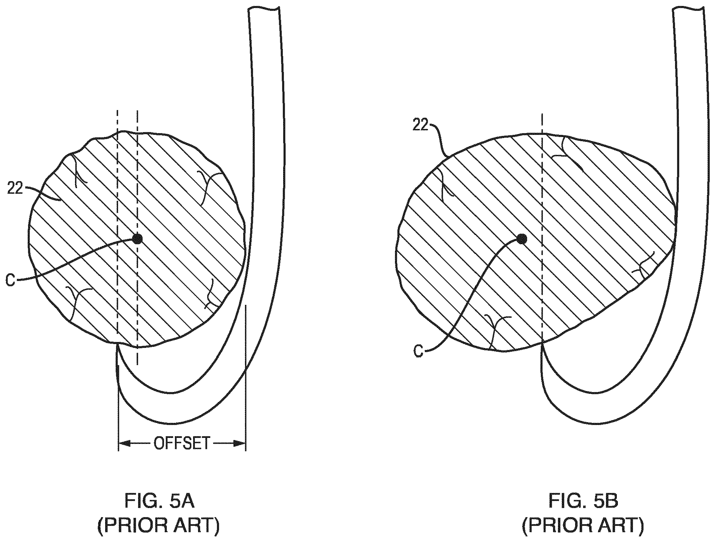

FIGS. 5A and 5B illustrate a guide distal aspect, known in the art. Guide may be coracoid guide disclosed in commonly owned US patent application number 2021/0015504, titled "Coracoid Drill Guide Assembly and Methods Of Use Thereof" and may include a guide arm with a hooked tip. The guide arm may engage a side surface of the coracoid 22, while the hooked tip may engage an inferior surface of the coracoid 22, as shown. The hook tip is laterally offset from the guide arm by a fixed offset distance. By engaging the hooked tip and arm, the drill trajectory may be guided through the coracoid 22, as illustrated. However, this distal aspect shown does not reliably center the drill trajectory, as illustrated. Centering depends on the actual curved outer surfaces of the coracoid cross section and the offset of the hook tip. For example, in FIG. 5A the hook offset is too great for the size and shape of the coracoid and places the drill trajectory off center. Another example is shown in FIG. 5B, where coracoid is more oblong in cross section, the oblong interacting with the example guide arm to place the drill trajectory off center. In practice the operator then needs to interpret and manually adjust the example guide that is known in the art to compensate for this off centering, taking both time, expertise and carrying with it some risk.

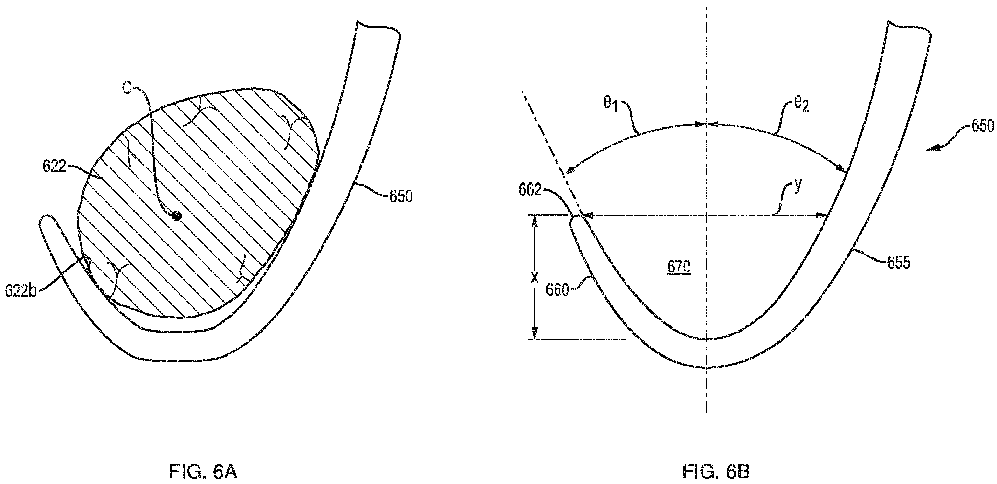

FIG. 6A illustrates a guide arm distal aspect 650 that has a fixed (non-adjustable) geometry, configured to center a passage trajectory through the coracoid 22, in accordance with this disclosure. The passage trajectory preferably extends from a first external surface to an opposing surface of the coracoid 22, defining a thickness or diameter of the bone. The term "diameter" is a straight-line distance through a bone anchor from one side of the bone to the other side of the bone that passes through a cross-sectional center of the bone. A "diameter" as used herein is not limited to an object with a circular cross-section. The passage trajectory is preferably from a superior surface to an inferior surface of the coracoid 22. The guide arm distal aspect 650 accommodates a range of curved outer surfaces of a coracoid, of varying diameters. The distal aspect 650 self-centers a drill passage trajectory despite engaging a rounded but not necessarily circular bone surface. The distal aspect 650 may be in the form a hook, shaped and configured as a rounded V-block. As such, the distal aspect 650 wraps around three sides of the coracoid 22 and engages it on two opposing sides (approximate anterior and posterior surfaces) of the coracoid 22. The distal aspect 650 wraps around the inferior/underside of the coracoid 22 but may remain spaced away from the inferior facing side, relying on the two opposing sides of contact to self-center the guide distal aspect 650 and therein the drill trajectory. Illustrated in FIG, 6A is an oblong coracoid cross section 622 engaged on two opposing sides by guide distal aspect 650 and may be spaced away from the inferior side. More specifically arm 655 extends from proximal aspect of guide and around a first side surface (anterior or posterior) of the coracoid 22. Arm distal aspect 650 engages coracoid on the first side surface 622a. Opposite the arm 655 is a free leg 660, terminating in a tip 662. Free leg 660 engages a second, opposing side surface 622b of the coracoid 22. Tip 662 may engage coracoid, depending on coracoid shape and curves.

At the distal aspect, arm 655 and free leg 660 define boundaries of a "V" or U" shaped opening 670, configured to receive the coracoid inferior surface therein. Opening 670 may define a maximum width "Y" configured to receive the coracoid 22 therethrough. Width "Y" may approximate a maximum cross section width or diameter of the target coracoid. Width "Y" may be between 12-18mm. Free leg 660 may extend in a retrograde direction and towards a guide proximal end defining an opening depth "X". Depth "X" is configured to receive the coracoid 22 inferior aspect therein sufficient to center the coracoid 22. Depth "X" may approximate half of the coracoid height or diameter when measured along a vertical plane (superior-inferior) of the target coracoid. Depth "X" may be between 6-9mm. Free leg 660 is limited in length for easy insertion through a small incision through the patient skin and around the inferior side of coracoid 22. The longer the free leg 660 the more rotation and/or translation may be required to place tip 662 around the inferior side of coracoid. This may require extra dissection around the coracoid 22, or a larger port size. "V" or U" shaped opening 670 may be symmetrical, extending at equal and opposite angles θ1, θ2 to each other. "V" or U" shaped opening 670 may define an angular opening that may be up to 120 degrees (°) in total. In some preferred embodiments, the opening 670 may define a 60-degree opening. Having a wider opening (closer to 120 degrees) may more easily slide under the coracoid. However, a wider opening may frustrate centering for small coracoid cross sections, as the inferior surface of coracoid 22 may preferably engage the distal aspect 650. As a reminder, guide distal aspect 650 preferably engages the inferior portion of coracoid 22 at two opposing sides of the coracoid 22 while remaining spaced away from the inferior side surface between these two opposing sides. This ensures that the two points of contact on the opposing sides center the guide distal aspect 650 around the coracoid 22. In some embodiments the distal aspect 650 may include gripping features such as teeth.

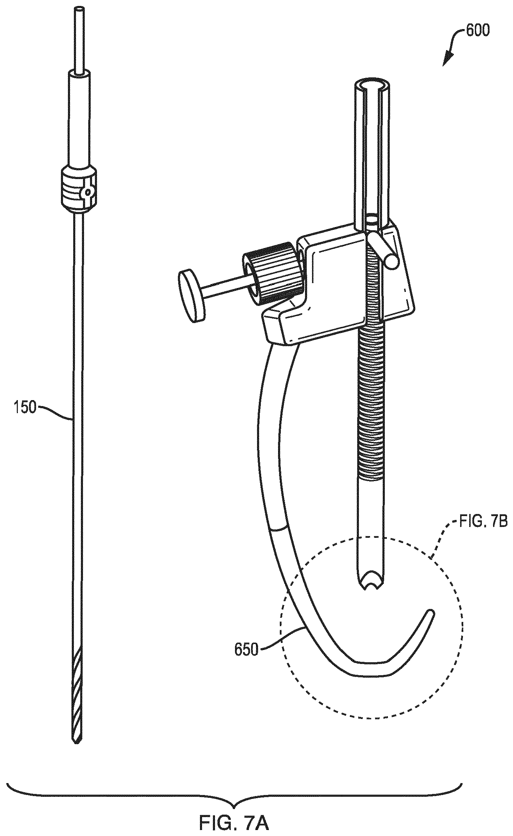

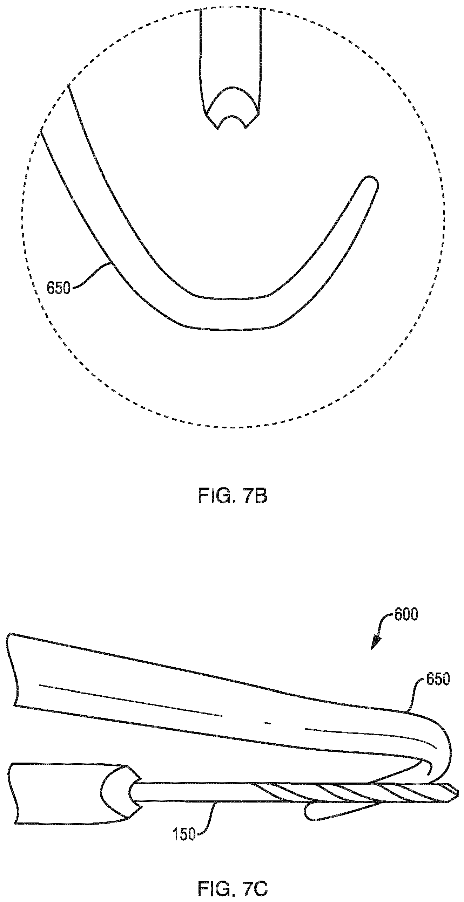

FIG. 7A illustrates a guide system 600 similar to system 100, with the exception of the guide distal aspect 650. FIG. 7B illustrates the guide system 600 with the drill 150 inserted through bullet, the trajectory of drill 150 offset from the distal aspect in a first direction and centered relative to the distal aspect 650 in a second, perpendicular direction.

A method of AC joint repair may include placing a distal aspect 650 of guide 600 around an inferior surface of the coracoid 22, the distal aspect 650 configured to center a coracoid cross section with respect to a drill trajectory. More specifically the distal aspect 650 may define a V-block opening and placing the distal aspect 650 places an inferior portion of the coracoid 22 within the V-block opening. Placing the distal aspect 650 may engage two opposing sides of the V-block opening with two opposing external surfaces of the coracoid 22, the two opposing sides approximately facing each other. Placing the distal aspect 650 may preferably avoid engagement of the inferior most surface of the coracoid. A bullet may then axially advance and engage a superior surface of clavicle 18. Guide may then be adjusted to hold the coracoid 22 and clavicle 18 in an anatomical reconstruction arrangement.

Bullet may be oriented to aim a drill trajectory through the clavicle 18 and coracoid 22. Drill 150 may be inserted through bullet to form a passage through the clavicle 18 and then through the coracoid 22, the passages axially aligned. The guide and bullet define a passage trajectory through both bones and disposes an inferior exit to the coracoid 22 at a posteriorly offset location along the coracoid 22 from the guide distal aspect 650. The guide and bullet define a passage trajectory through both bones and disposes a passage trajectory through the coracoid 22 through the center of the coracoid 22. Drill 150 may then be removed, while the guide 600 and bullet remain engaged with the two bones. While the guide remains engaged, a repair construct may be operatively coupled to the two bones. The repair construct may include a coracoid anchor, placed at the inferior passage exit while guide remains engaged with the two bones.

One skilled in the art will realize the disclosure may be embodied in other specific forms without departing from the essential characteristics thereof. The foregoing examples are therefore to be considered in all respects illustrative rather than limiting of the disclosure described herein. Scope of the disclosure is thus indicated by the appended claims, rather than by the foregoing description, and all changes that come within the meaning and range of equivalency of the claims are therefore intended to be embraced therein.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

## Claims

1. An offset guide (100) for holding a surgical joint in a reduced configuration comprising:
a proximal end (120), a distal end (110) and a curved arm (130) extending therebetween;
wherein the distal end is configured to engage a bone of the surgical joint at a first location along the bone and apply a load to the bone to hold the bone in the reduced configuration;
wherein the proximal end includes a guide sleeve (170) configured to guide placement of a surgical tool through the bone towards an offset location along the bone, the offset location spaced away from the first location,
**characterised in that** the guide distal end defines a hook configured to wrap around three sides of the bone and self-center the distal end relative to the bone such that the surgical tool is placed through a center of the bone at the offset location

2. The offset guide of claim 1, wherein the offset guide comprises a bullet (115) receivable in the guide sleeve, the bullet having a distal end (118) configured to engage a superior surface of a clavicle (18).

3. The offset guide of claim 1 or 2, wherein the distal end of the curved arm defines a finger (111) defining a proximal facing surface configured to wrap around and engage an inferior surface of the coracoid (22).

4. The offset guide of claim 1, 2, or 3 wherein the surgical tool includes a drill tool (150) and an implant insertion tool.

5. The offset guide of claim 1, 2, or 3 wherein the distal end is offset by at least 5mm from the offset location.

6. The offset guide of claim 1, 2, or 3 wherein the guide distal end is configured to engage an apex (123) of a coracoid bone.

## Patentansprüche

1. Versatzführung (100) zum Halten eines chirurgischen Gelenks in einer reduzierten Konfiguration, die Folgendes umfasst:
ein proximales Ende (120), ein distales Ende (110) und einen gekrümmten Arm (130), die sich dazwischen erstreckt,
wobei das distale Ende dazu ausgestaltet ist, einen Knochen des chirurgischen Gelenks an einer ersten Stelle entlang des Knochens in Eingriff zu nehmen und eine Belastung auf den Knochen auszuüben, um den Knochen in der reduzierten Konfiguration zu halten,
wobei das proximale Ende eine Führungshülse (170) aufweist, die dazu ausgestaltet ist, die Platzierung eines chirurgischen Werkzeugs durch den Knochen zu einer versetzten Stelle entlang des Knochens zu führen, wobei die versetzte Stelle von der ersten Stelle beabstandet ist,
**dadurch gekennzeichnet, dass** das distale Führungsende einen Haken definiert, der dazu ausgestaltet ist, drei Seiten des Knochens zu umgreifen und dafür zu sorgen, dass sich das distale Ende selbst bezüglich des Knochens zentriert, so dass das chirurgische Werkzeug durch eine Mitte des Knochens an der versetzten Stelle platziert wird.

2. Versatzführung nach Anspruch 1, wobei die Versatzführung einen Kugelkopf (115) umfasst, der in der Führungshülse aufgenommen werden kann, wobei der Kugelkopf ein distales Ende (118) aufweist, das zur Ineingriffnahme einer oberen Fläche eines Schlüsselbeins (18) ausgestaltet ist.

3. Versatzführung nach Anspruch 1 oder 2, wobei das distale Ende des gekrümmten Arms einen Finger (111) definiert, der eine proximale zugewandte Fläche definiert, die dazu ausgestaltet ist, eine untere Fläche des Coracoids (22) zu umgreifen und diese in Eingriff zu nehmen.

4. Versatzführung nach Anspruch 1, 2 oder 3, wobei das chirurgische Werkzeug ein Bohrwerkzeug (150) und ein Implantateinführungswerkzeug aufweist.

5. Versatzführung nach Anspruch 1, 2 oder 3, wobei das distale Ende um mindestens 5 mm von der Versatzstelle versetzt ist.

6. Versatzführung nach Anspruch 1, 2 oder 3, wobei das distale Führungsende zur Ineingriffnahme eines Scheitelpunkts (123) eines Coracoidknochens ausgestaltet ist.

## Revendications

1. Guide décalé (100) destiné à maintenir une articulation chirurgicale dans une configuration réduite comprenant :
une extrémité proximale (120), une extrémité distale (110) et un bras incurvé (130) s'étendant entre elles ;
dans lequel l'extrémité distale est configurée pour entrer en contact avec un os de l'articulation chirurgicale à un premier emplacement le long de l'os et pour appliquer une charge à l'os afin de maintenir l'os dans la configuration réduite ;
dans lequel l'extrémité proximale comporte un manchon de guidage (170) configuré pour guider le placement d'un instrument chirurgical à travers l'os vers un emplacement décalé le long de l'os, l'emplacement décalé étant espacé du premier emplacement,
**caractérisé en ce que** l'extrémité distale du guide définit un crochet configuré pour s'enrouler autour de trois côtés de l'os et autocentrer l'extrémité distale par rapport à l'os de telle sorte que l'outil chirurgical soit placé à travers un centre de l'os à l'emplacement décalé.

2. Guide décalé selon la revendication 1, dans lequel le guide décalé comprend une cartouche (115) pouvant être reçue dans le manchon de guidage, la cartouche ayant une extrémité distale (118) configurée pour entrer en prise avec une surface supérieure d'une clavicule (18).

3. Guide décalé selon la revendication 1 ou 2, dans lequel l'extrémité distale du bras incurvé définit un doigt (111) définissant une surface tournée de façon proximale configurée pour s'envelopper autour d'une surface inférieure du coracoïde (22) et entrer en prise avec celle-ci.

4. Guide décalé selon la revendication 1, 2 ou 3, dans lequel l'outil chirurgical comporte un outil de perçage (150) et un outil d'insertion d'implant.

5. Guide décalé selon la revendication 1, 2 ou 3, dans lequel l'extrémité distale est décalée d'au moins 5 mm par rapport à l'emplacement décalé.

6. Guide décalé selon la revendication 1, 2 ou 3, dans lequel l'extrémité distale du guide est configurée pour entrer en prise avec un sommet (123) d'un os coracoïde.
